# EUROPEAN PATENT APPLICATION

(11) **EP 1 990 641 A1**
(43) Date of publication of application: **12.11.2008**
(21) Application number: 07108043.6
(22) Date of filing: 11.05.2007
(51) Int. Cl.: G01N 35/02, G01N 33/50, B01L 3/00

(54) **Flow-through biosensor device**

(71) Applicant: Koninklijke Philips Electronics N.V., 5621 BA Eindhoven (NL)
(72) Inventor: The designation of the inventor has not yet been filed
(74) Representative: Van Velzen, Maaike Mathilde

(57) **Abstract**

The present invention provides a diagnostic device for detecting an analyte in a fluid comprising a sealed cell. A porous membrane comprising a capture probe is arranged inside the cell and divides the cell into two compartments. An elastic fluid-tight membrane which forms at least part of a sidewall of one of the compartments is used to pump a fluid present in the cell through the porous membrane from one compartment to the other. When the elastic membrane is deformed and, consequently, the volume of one of the compartments is changed, the fluid is pumped through the porous membrane. The other compartment into which the fluid is pumped is adapted to receive the fluid. Furthermore, a method for detecting an analyte in a fluid using a device according to the present invention is provided.

## Description

### FIELD OF THE INVENTION

The present invention relates to a diagnostic device for detecting the presence of an analyte in a sample, wherein the sample flows through a porous membrane comprising a capture probe.

### BACKGROUND OF THE INVENTION

Devices for molecular diagnostics are generally built around substrates that contain patterns of capture probes for detecting the presence of specific analytes in a sample. The capture probes generally comprise receptor molecules which are capable of binding with biomolecular species of the analyte to be detected, such as nucleic acids, proteins, cells or drugs present within the sample. For example, for detecting whether an antigen is present in the sample, antibody binding may be used. For detecting the presence of DNA, protein interactions may be applied.

Various detection methods have been developed to sense whether the specific binding has occurred. Commonly, an optical detection method is used that is based on, for example, fluorescence, refractive index changes or spectral changes. Also changes in localized magnetic or dielectric behaviour are being used. In a particular example of an optical capture probe, a membrane substrate is provided with an array of capture targets of different receptor molecules, for example applied via inkjet printing and/or contact printing. The receptor molecules are brought into contact with the sample containing the analyte via a direct contact in a well plate or in a micro-channel using a so-called flow-over principle. In order to enhance the sensitivity of the detection device and for reducing the assay time needed to diagnose the sample, a flow-through principle may alternatively be used.

In order to be detected, the biomolecules of interest that is the analytes, have to bind specifically to the corresponding capture probes. This step is often referred to as either hybridization, in case of a DNA analyte, or incubation, in case of a protein analyte. For determining whether the specific capturing took place, fluorescent labeled ssDNA fragments or antibodies may be used which will bind to the capture probe:ligand complex. The presence of the specific biomolecules can then be detected by means of an optical detection device, such as a CCD camera if the fluorescent probes are excited to emit light.

In a device which works according to the flow-over concept mentioned above, the sample comprising the analyte to be detected flows parallel to a solid substrate which comprises an array ofnanowells in which the capture molecules are located. Usually, the spots comprising the capture molecules are printed in series such that the analyte may hit every capture probe. However, since the flow of the sample in the micro fluidic device normally is laminar, the transport of the analyte to the capture sites should in practice occur by diffusion. This results in long assay times if the channel depth is large in comparison to the sizes of the molecules to be detected, which is normally the case. Flow-over devices furthermore generally have a rather low sensitivity due to the limitation of the number of capture probes per projected area.

In a flow-through device, the analyte flows perpendicular through a microporous substrate, such as a membrane comprising immobilized capture molecules. The high specific surface area of the microporous substrate causes a large number of specifically bonded probe molecules on the detection spots, resulting in a high sensitivity for the detection of bonded molecules. One of the main advantages of flow-through devices in comparison with flow-over devices is that flow-through devices are not diffusion dependent. Due to the high specific surface area of the microporous substrate, the detection spots comprise a large number of specifically bonded probe molecules, resulting in a high sensitivity for the detection of the analyte.

In US-A-2005/0191620, an analyte detection system for both membrane and/or sensor array particle-based measurements is disclosed which may be used to determine the presence of analytes. In a membrane based flow sensor disclosed in this reference, a flow sensor includes a membrane that is sandwiched between at least two members which are configured to allow fluids to flow to and through a membrane. The membranes are also configured to allow detection of analytes, after the analytes have been captured on the membrane.

Other flow-through devices are described in WO2005/077537, WO2000/20864, US-A-5 038 793 and US-B-6 852 289, the latter describing combining several flow-through modules in one apparatus for simultaneous processing of any desired member of samples.

However, flow-through devices generally require larger volumes of the sample comprising the analyte, in particular if the sample comprises a low concentration of the analyte since the capture area on the substrate through which the sample flows is placed parallel to the flow of the sample and therefore, the specific targeted area misses many analyte molecules as they pass outside this targeted area.

### SUMMARY OF THE INVENTION

It is an object of the present invention to provide a diagnostic device using the flow-through technique having a simple construction and improved robustness. The detection device should further require a low sample volume while providing a sensitivity compared to the known systems.

In order to achieve these objects, the present invention provides a diagnostic device for detecting an analyte in a fluid comprising a sealed cell. A porous membrane comprising a capture probe is arranged inside the cell and divides the cell into two compartments. An elastic fluid-tight membrane which forms at least part of a sidewall of one of the compartments is used to pump a fluid present in the cell through the porous membrane from one compartment to the other. When the elastic membrane is deformed, e.g. by exerting a force on the elastic membrane, and, consequently, the volume of one of the compartments is changed, the fluid is pumped through the porous membrane. The other compartment into which the fluid is pumped is adapted to receive the fluid. For compensating for the volume change in the other compartment due to the addition of fluid, also a side wall of the other compartment may be formed by another elastic membrane. Alternatively, it may be possible to connect the other compartment to a reservoir.

Depending on the deformation of the elastic membrane, the flow of the fluid may be performed through the porous membrane in two opposite directions. For example, by pressing onto the elastic membrane, the fluid may be pressed from the first compartment of which the elastic membrane forms part of a sidewall to the second compartment. Accordingly, by exerting a negative pressure on the elastic membrane, the fluid may be sucked back from the second compartment to the first compartment.

Furthermore, by providing two elastic membranes, each one forming at least part of a sidewall of one of the compartments, respectively, the fluid may consecutively be pumped through the porous membrane in opposite directions by alternately deforming the first and the second elastic membranes.

According to an embodiment of the invention, the porous membrane is arranged on a holder which can easily be placed into the cell and taken out of the cell. Depending on the desired tests, the diagnostic device may comprise a plurality of sealed cells each one comprising a porous membrane and an elastic membrane forming a sidewall of each of these cells, respectively. In that way, an array of test cells is formed wherein several tests may be carried out simultaneously.

Furthermore, a method for detecting an analyte in a fluid using a device according to the present invention is provided.

These and other aspects of the invention will be apparent from and elucidated with reference to the embodiments described hereinafter.

### BRIEF DESCRIPTION OF THE DRAWINGS

Figure 1 schematically shows the cell of a diagnostic device according to a preferred embodiment of the present invention.
Figure 2 shows an exploded view of a diagnostic device according to an embodiment of the present invention.
Figure 3 shows a perspective view of a part of the diagnostic device according to the embodiment shown in Figure 2.
Figure 4 shows a perspective view of the assembled diagnostic device according to the embodiment shown in Figure 2.
Figure 5 shows a perspective view of a diagnostic device according to an embodiment of the present invention including several cells.
Figure 6 shows the comparison of intensities of a measurement performed without pumping and two measurements with pumping the fluid through the membrane.
Figure 7 shows a comparison between a standard incubation and flow-through.
Figure 8 shows a dose response curve performed without and with pumping
Figure 9 shows the binding kinetics of an analyte using pumping or without pumping.

### DETAILED DESCRIPTION OF EMBODIMENTS

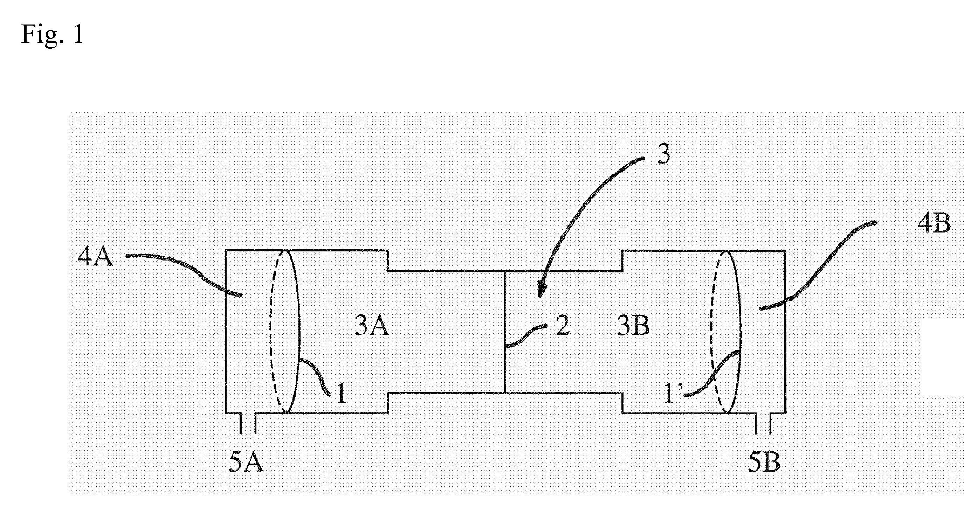

Figure 1 schematically shows the sealed cell of a diagnostic device according to an embodiment of the present invention in order to illustrate the basic principle used for pumping the fluid comprising the analyte to be detected through a porous membrane.

As shown in Figure 1, the porous membrane 2 divides the cell 3 to form two separate compartments 3A and 3B. One sidewall of each compartment 3A and 3B is formed by an elastic membrane 1 and 1', respectively. As indicated in Figure 1, porous membranes 1, 1' may be deformed in a way such that the volume of the compartments 3A and 3B may be increased or decreased.

Figure 1 further illustrates a driving mechanism to deform the elastic membranes 1 and 1'. In the embodiment shown in Figure 1, the driving mechanism comprises additional compartments 4A and 4B which are separated from the cell 3 by the elastic membranes 1 and 1'. By pumping a pressurized gas or liquid into one of the two compartments 4A or 4B via connections 5A and 5B, a force is exerted on one of the elastic membranes 1 or 1'. Consequently, due to the deformation of the membrane, the volume of one of compartments 3A and 3B is decreased and a portion of the fluid present in the compartment which is made smaller is pumped through the porous membrane 2 to the other compartment.

By alternately pumping a pressurized gas or liquid into compartments 4A and 4B, the fluid is pumped consecutively through the porous membrane in opposite directions.

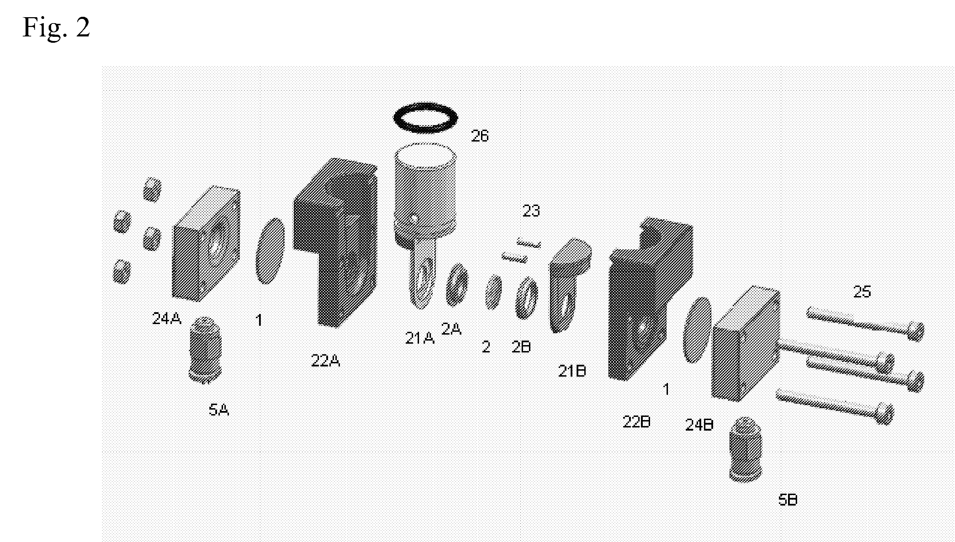

A diagnostic cell according to an embodiment of the present invention in an exploded view is shown in Figure 2. The porous membrane 2 is fixed by fixing rings 2A and 2B. Parts 21A and 21B act as a holder for the membrane. In order to place the porous membrane in the holder, first membrane 2 fixed by fixing rings 2A and 2B is placed on part 21A. Part 21B is then used to lock the holder, guided by bolts 23.

The sealed cell of the diagnostic device is formed by parts 22A and 22B. Elastic membranes 1 and 1' which form opposite sidewalls of the cell are held by parts 24A and 24B which are fixed by screws 25.

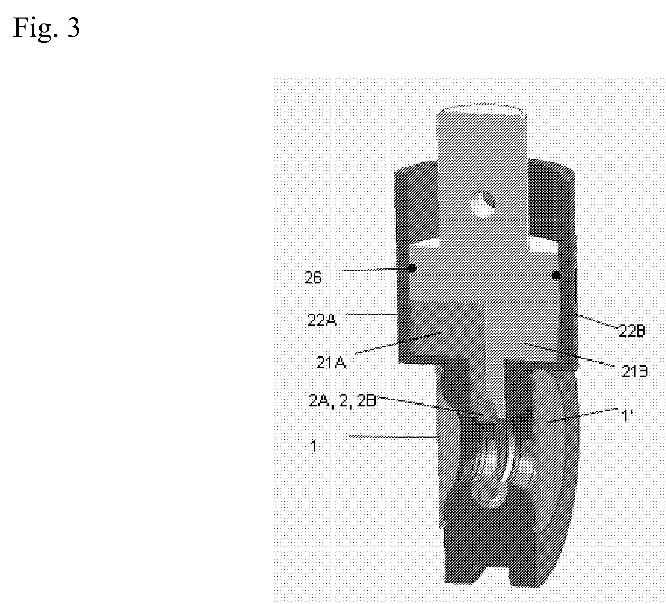

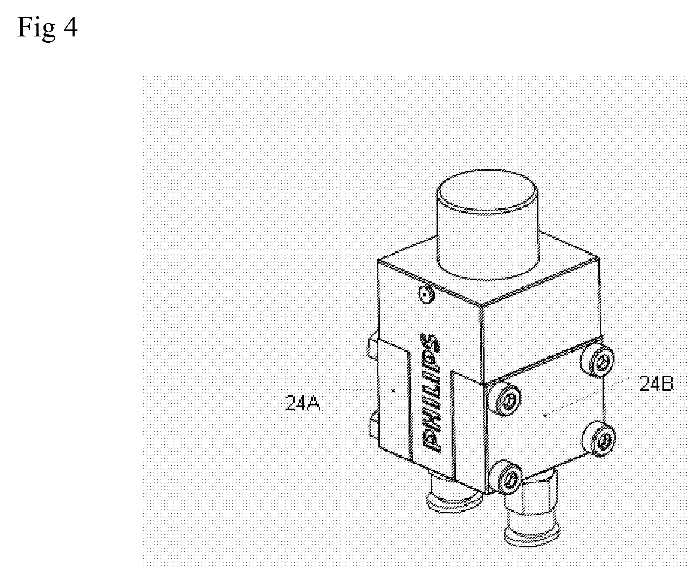

In order to fill the fluid which comprises the analyte to be detected into the cell, the holding structure comprising parts 21A, 2A, 2B and 21B holding the porous membrane 2, may be removed and placed back into the cell. In order to seal the cell, a seal ring 26 is provided. Fig. 3 shows the diagnostic cell shown in Fig. 2 partly assembled, omitting parts 24A and 24B. In Fig. 4, the fully assembled diagnostic device is shown in a perspective view.

Thus, the diagnostic device of the embodiment shown in Figures 2 to 4 comprises a specially designed cylindrical opening forming the cell which on both sides is hermetically sealed with the elastic membrane. The holding structure designed for holding the porous membrane is easy to take out and place back into the cylindrical opening. The handling of the holding structure may be performed manually or may alternatively be fully automated.

Upon filling the fluid to be tested into the cell and placing the holding structure with the porous membrane into the cell, the fluid to be tested is about equally distributed to the two compartments of the cell, and the elastic membranes 1 and 1' are in a neutral position, as shown in Figure 3. In this position, both compressed air connections 5A and 5B are open to the atmosphere. By pumping compressed air through one of the air connections, the respective elastic membrane is pushed inwards into the cell, thus pressing almost all fluid present in the respective compartment to the other compartment through the porous membrane. Then, compressed air is pumped through the other air connection, forcing the other elastic membrane to move inwards, pushing all the fluid through the porous membrane back to the original compartment. By alternately applying compressed air on connections 5A and 5B, an alternating movement of the fluid through the porous membrane in opposite directions is achieved.

After the desired number of movements, the mechanism for applying compressed air connection is switched off and the system returns to the neutral position. The membrane may then be analyzed either using a detection system integrated to the diagnostic device or by removing the holder and placing the porous membrane into a separate detection system.

Even though with reference to Figure 1 to 4, embodiments of the diagnostic device of the present invention have been described using compressed air to deform the elastic membrane, other driving mechanisms may be provided. For example, it is possible to use a mechanical driving mechanism using, for example, a motor with a rotating excenter to create a linear motion that operates on the cell membrane either directly or indirectly. Also an electromechanical mechanism may be used wherein by applying a current through a core is moved inwards and outwards of the core acting on the elastic membrane.

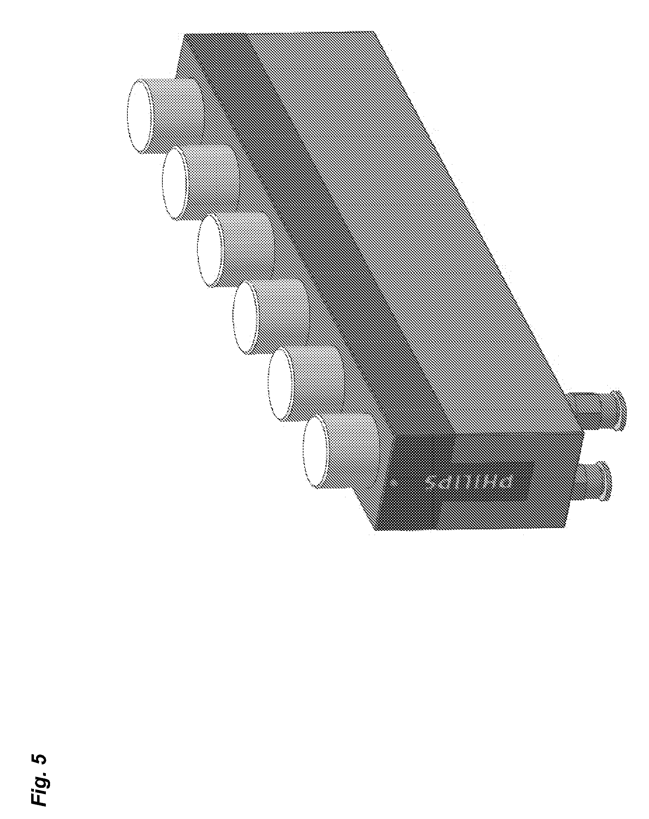

According to an embodiment of the invention, a plurality of sealed cells may be provided in a single diagnostic device. The plurality of cells may be provided in a linear arrangement, as shown in Figure 5. Alternatively, also a circular arrangement is possible. The driving mechanism may be arranged in a way to simultaneously act on the elastic membranes of all cells. For example, in the above described embodiment of the invention which uses compressed air to deform the elastic membrane, compartments 4A and 4B shown in Figure 1 may extend along all cells, so that pumping compressed air into one of the compartments 4A or 4B simultaneously deforms all membranes connected to this compartment.

The diagnostic device of the present invention has been used to compare the reaction speed of binding of an antibody with protein printed on the porous membrane, when performing repeated pump cycles or no pump cycles at all. To this end, 500 µl of a 100nM Cy5 labeled Rabbit-anti-Mouse antibody solution was placed in the device. The membrane was put in the liquid filled device. The solution was pumped approximately 10 times in 30 seconds. After these 30 seconds the membrane was washed 3 times 5 minutes with 1xPBS+0.05%Tween-20 to remove unbound and aspecific bound antibodies. Then a fluorescent image was taken of the membrane. After this step the device was cleaned and a new 100nM Cy5 labeled Rabbit-anti-Mouse solution was added. This time, however, there was no pumping within the 30 seconds. After these 30 seconds the membrane was washed and imaged using the same protocol as the previous mentioned membrane.

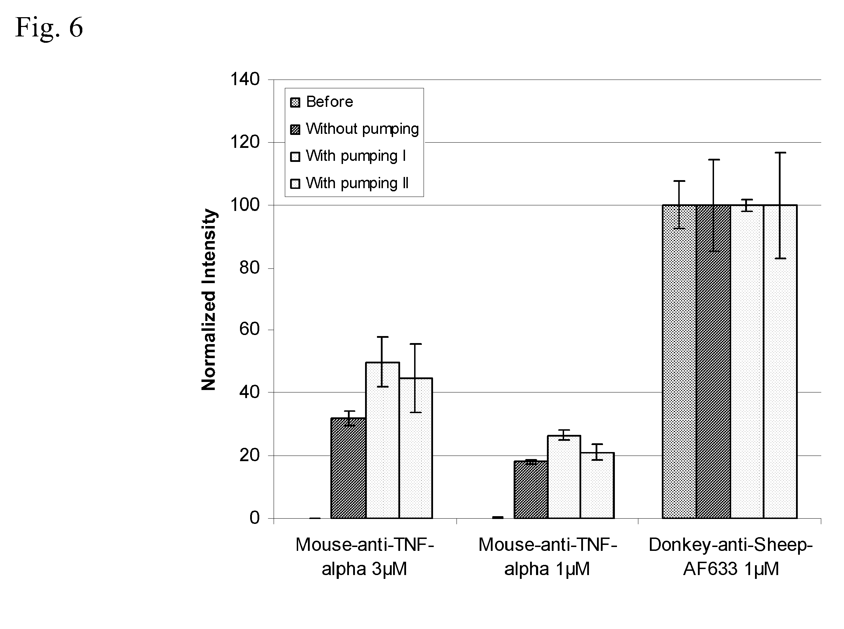

The intensities of the different measurements are shown in Fig. 6. Comparing the intensity coming from the Cy5 labeled Rabbit-anti-Mouse antibody it is clear that the pumping has a positive effect on the reaction seed and therefore, the flow-through takes place. This effect is higher when printing higher amounts of antibodies (i.e. 4µM). Also it is to be expected that the difference between pumping and no pumping increases in time. Although it has to be noted that the pumping does not affect the binding efficiency of the antibodies and therefore, it has no effect on the maximum binding capacity.

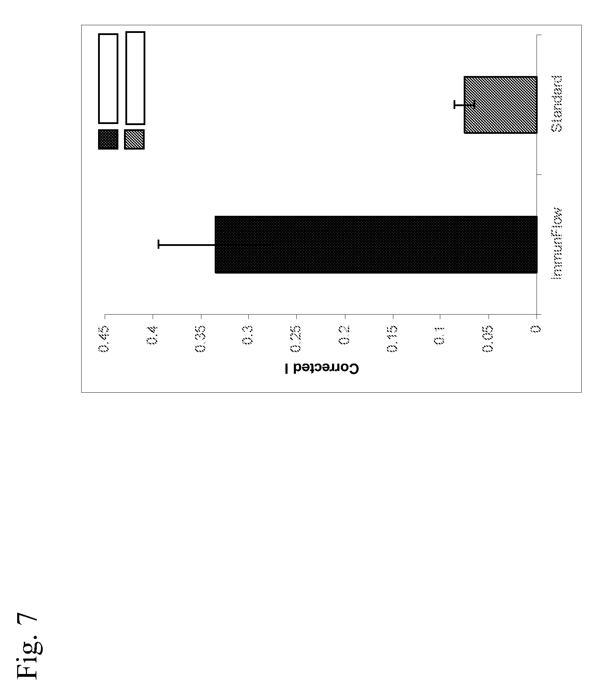

Figure 7 shows the comparison between a standard incubation (e.g. no flow) and flow-through. The 500µl of analyte, Rabbit-anti-Mouse-AF633, with a concentration of 13.3 pM is incubated with and without pumping for 30 minutes. After these 30 minutes the membrane was washed 3 times 5 minutes with 1xPBS + 0.05% Tween-20 to remove unbound and aspecificly bound analytes. Then a fluorescent image was taken of the membrane.

The intensities of the different measurements are shown in Fig. 7. Comparing the intensity originating from the AF633 labeled Rabbit-anti-Mouse antibody it is clear that the pumping has a positive effect on the reaction speed and therefore, the flow-through takes place.

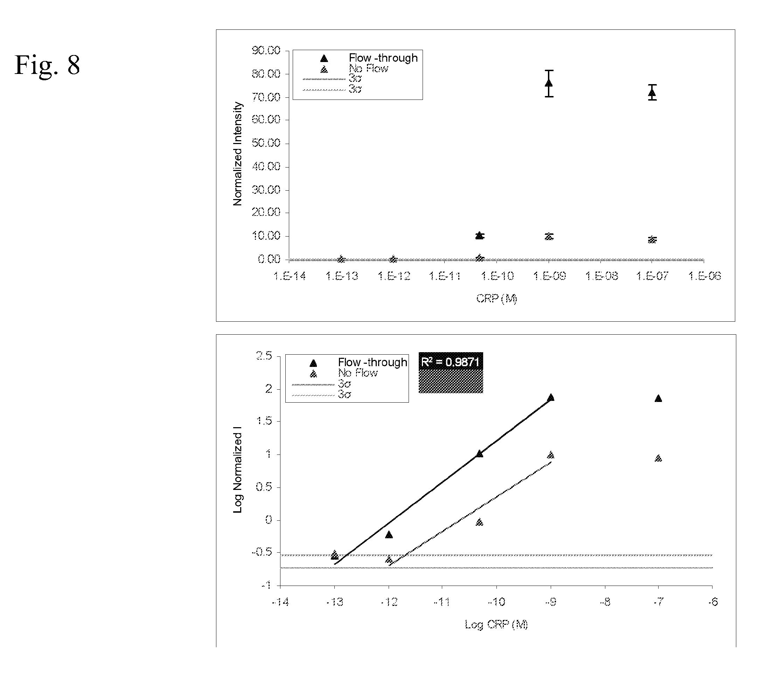

The results described in Figures 6 and 7 are performed using an immunoassay where the analyte itself can be detected. A commonly used immunoassay is the sandwich assay which is tested in Figure 8. In this embodiment the analyte is bound to the capture probe via incubation for 60 minutes with and without pumping. Then the membrane is washed 3 times 5 minutes with 1xPBS+0.05%Tween-20 to remove unbound and a specific bound antibodies. The analyte, that is bound, is not visible using fluorescent techniques. Therefore, a biotin labeled secondary antibody is incubated for 60 minutes with and without pumping and again the membrane is washed. The last step consists of Streptavidin labeled with AF647 which will bind to biotin. The AF647 labeled Streptavidin is incubated for 30 minutes with and without pumping. After these 30 minutes the membrane was washed 3 times 5 minutes with 1xPBS + 0.05% Tween-20 to remove unbound and aspecific bound analytes. Then a fluorescent image was taken of the membrane.

The intensities of the different measurements are shown in Fig. 8. Comparing the intensity coming from the analyte-detection-antibody-Streptavidin-AF647 complex it is clear that the pumping has a positive effect on the signal intensity. The effect is depending on the analyte concentration as to be expected looking at the reaction kinetics. Furthermore the detection limit is increased when using flow-through.

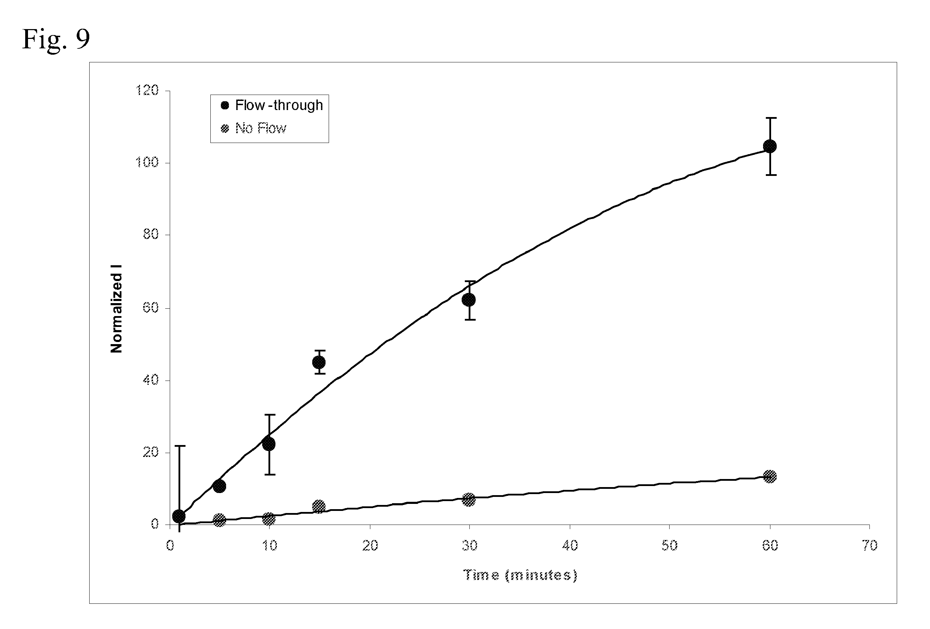

Figure 9 shows the kinetics between the capture probe and the analyte. Again a sandwich Immunoassay is performed where the analyte (500µl 100pM) incubation step is performed varying the incubation time. After this incubation the membrane was washed 3 times 5 minutes with 1xPBS + 0.05% Tween-20 to remove unbound and aspecific bound analytes. Then all of the membranes are treated in the same way. A biotin labeled secondary antibody is incubated for 60 minutes without pumping and again the membrane is washed. The last step consist of Streptavidin labeled with AF647 which will bind to biotin. The AF647 labeled Streptavidin is incubated for 30 minutes without pumping. After these 30 minutes the membrane was washed 3 times 5 minutes with 1xPBS + 0.05% Tween-20 to remove unbound and aspecific bound analytes. Then a fluorescent image was taken of the membrane.

The binding kinetics are much faster for the flow-through. Also the amount of analyte bound to the capture antibody is sufficient after 5 minutes to be detected. However, to detect it there is a need for some extra steps that take time (circa 120 minutes). It is to be expected that this can be greatly reduced by optimizing the system.

## Claims

1. Diagnostic device for detecting an analyte in a fluid, comprising:
a sealed cell;
a porous membrane(2) comprising a capture probe for detecting said analyte, said porous membrane (2) being arranged in said cell so as to divide said cell to form a first and a second separate compartment (3A and 3B, respectively);
an elastic, fluid-tight membrane (1) forming at least part of a side wall of the first compartment (3A) of said cell; and
means adapted to deform the elastic membrane (1) for changing the volume of the first compartment (3A) and to pump the fluid through the porous membrane (2) from the first compartment (3A) to the second compartment (3B) or vice versa, the second compartment (3B) being adapted to receive the fluid.

2. Device according to claim 1, further comprising a second elastic, fluid-tight membrane (1') forming at least part of a side wall of the second compartment (3B) of the cell.

3. Device according to claim 2, further comprising a second means adapted to deform the second elastic membrane (1), for changing the volume of the second compartment (3B) and to pump the fluid through the porous membrane (2) from the second compartment (3B) to the first compartment (3A) or vice versa.

4. Device according to claim 3, wherein by alternately deforming the first and the second elastic membranes (1 and 1', respectively), the fluid is pumped consecutively through the porous membrane (2) in opposite directions.

5. Device according to claim 1, wherein said means adapted to deform the elastic membrane (1, 1') comprises an additional compartment (4A, 4B) sealed from the cell by the elastic membrane (1, 1') and means adapted to pump a gas or a liquid in the additional compartment (4A, 4B).

6. Device according to claim 1, wherein said elastic membrane (1, 1') is a silicone membrane.

7. Device according to claim 1, wherein said porous membrane (2) is arranged on a holder (21A, 2A, 2B, 21B) which is removable fixed in said cell.

8. Device according to claim 1, further comprising a detection device for diagnosing the capture probe.

9. Device according to claim 1, wherein said analyte is at least one of a nucleic acid, a protein, a cell, and a drug.

10. Detection device according to claim 1, said device comprising a plurality of said sealed cells wherein said means adapted to deform the elastic membrane (1, 1') is adapted to deform the membranes of each of the cells simultaneously.

11. Method for detecting an analyte in a fluid using a device according to claim 1 comprising the following steps:
applying the fluid to the cell;
pumping the fluid through the porous membrane (2) by deforming the elastic membrane (1);
removing the porous membrane (2) from the cell; and
determining whether the analyte has been detected by the capture probe on the porous membrane (2).

12. Method according to claim 11, wherein the fluid is alternately pumped through the porous membrane (1) a predetermined number of times from one compartment to the other compartment in opposite directions.

13. Method according to claim 12, wherein the fluid is pumped at least 10 times.
